(19) Europäisches Patentamt
European Patent Office
Office européen des brevets

(11) Veröffentlichungsnummer: **0 000 538**
**A1**

(12) **EUROPÄISCHE PATENTANMELDUNG**

(21) Anmeldenummer: 78100425.4

(22) Anmeldetag: 18.07.78

(51) Int. Cl.$^2$: C 07 D 487/04, A 61 K 31/55 //C07D239/91, (C07D487/04, 243/00, 239/00)

(30) Priorität: 26.07.77 DE 2733681
30.12.77 DE 2758875

(43) Veröffentlichungstag der Anmeldung: 07.02.79 Bulletin 79/3

(84) Benannte Vertragsstaaten: BE CH DE FR GB LU NL SE

(71) Anmelder: Bayer Aktiengesellschaft
Zentralbereich Patente,
Marken und Lizenzen Bayerwerk
D-5090 Leverkusen 1. (DE)

(72) Erfinder: Mayer, Karl Heinrich, Dr.
Bürgerbuschweg 100
D-5090 Leverkusen 3. (DE)

(72) Erfinder: Heitzer, Helmut, Dr.
Höhenstrasse 84
D-5090 Leverkusen 3. (DE)

(72) Erfinder: Hoffmeister, Friedrich, Prof.Dr.
Katernberger Strasse 262
D-5600 Wuppertal 1. (DE)

(72) Erfinder: Heise, Arend, Dr
Möbeck 50
D-5600 Wuppertal 11. (DE)

(72) Erfinder: Kazda, Stanislav, Dr.
Pahlkestrasse 55N
D-5600 Wuppertal 1. (DE)

(54) **6,7-Dihydro-5H,13H-chinazolino(3,2-a)(1,4)benzodiazepin-5,13-dione, Verfahren zu ihrer Herstellung sowie ihre Verwendung als Arzneimittel.**

(57) 6,7-Dihydro-5H,13H- chinazolino (3,2-a)(1,4) benzodiazepin- 5,13-dione der allgemeinen Formel (I)

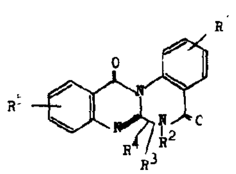

(I)

in welcher

$R^1$ und $R^5$ für 1,2,3 oder 4 Substituenten aus der Gruppe Wasserstoff, Alkyl, Hydroxy, Acyloxy, Alkoxy Nitro, Amino, Alkylamino, Dialkylamino, Acylamino, Acylalkylamino, Alkoxycarbonylamino Halogen, Trifluoromethyl, Cyan, Carboxy, Alkoxycarbonyl, Aminocarbonyl, Alkylaminocarbonyl, Dialkylaminocarbonyl, Aminosulfonyl, Alkylaminosulfonyl, Dialkylaminosulfonyl, Alkylaminocarbonylamino und Dialkylaminocarbonylamino steht,

$R^2$ für Wasserstoff, gegebenenfalls substituiertes Alkyl, Aralkyl, Aryl oder für Heterocycloalkyl, Acyl oder Alkoxycarbonyl steht und

$R^3$ und $R^4$ für gleiche oder verschiedene Substituenten aus der Gruppe Wasserstoff, gegebenenfalls substituiertes Alkyl, gegebenenfalls im Arylteil substituiertes Aralkyl, gegebenenfalls substituiertes Aryl oder Heteroaryl steht

sowie ihre Verwendung als Arzneimittel, vorzugsweise als auf das Zentralnervensystem wirkende Stoffe, insbesondere als cerebralatiischämische, antiamnetische, lern-, leistungsund gedächtnisverbessernde, tranquillisierende, analgetische und antipyretische Wirkstoffe.

Die Verbindungen werden durch Umsetzung eines 2-Alkyl-3- aryl-4H- chinazolin-4-ons mit $H_2N$-$R^2$ oder $NH_3$ hergestellt

EP 0 000 538 A1

BAYER AKTIENGESELLSCHAFT
Zentralbereich
Patente, Marken und Lizenzen

5090 Leverkusen, Bayerwerk
KS/bc
Ia (Pha)

6,7-Dihydro-5H,13H-chinazolino[3,2-a][1,4]benzodiazepin-5,13-dione, Verfahren zu ihrer Herstellung sowie ihre Verwendung als Arzneimittel

Die vorliegende Erfindung betrifft neue 6,7-Dihydro-5H,13H-chinazolino[3,2-a][1,4]benzodiazepin-5,13-dione, ein Verfahren zu ihrer Herstellung sowie ihre Verwendung als Arzneimittel, vorzugsweise als auf das Zentralnervensystem wirkende Stoffe, insbesondere als cerebralantiischämische, antiamnetische, lern-, leistungs- und gedächnisverbessernde, tranquillisierende, analgetische und antipyretische Wirkstoffe.

Die Erfindung betrifft neue 6,7-Dihydro-5H,13H-chinazolino[3,2-a][1,4]benzodiazepin-5,13-dione der allgemeinen Formel (I)

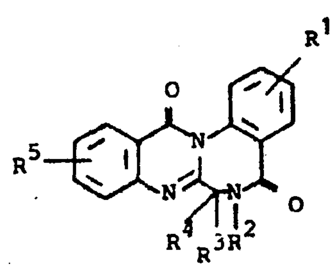

(I)

in welcher

$R^1$ und $R^5$ für 1, 2, 3 oder 4 Substituenten aus der Gruppe Wasserstoff, Alkyl, Hydroxy, Acyloxy, Alkoxy, Nitro, Amino, Alkylamino, Dialkylamino, Acylamino, Acylalkylamino, Alkoxycarbonylamino, Halogen, Trifluormethyl, Cyan, Carboxy, Alkoxycarbonyl, Aminocarbonyl, Alkylaminocarbonyl, Dialkylaminocarbonyl, Aminosulfonyl, Alkylaminosulfonyl, Dialkylaminosulfonyl, Alkylaminocarbonylamino und Dialkylaminocarbonylamino steht,

$R^2$ für Wasserstoff, gegebenenfalls substituiertes Alkyl, Aralkyl, Aryl oder für Heterocycloalkyl, Acyl oder Alkoxycarbonyl steht und

$R^3$ und $R^4$ für gleiche oder verschiedene Substituenten aus der Gruppe Wasserstoff, gegebenenfalls substituiertes Alkyl, gegebenenfalls im Arylteil substituiertes Aralkyl, gegebenenfalls substituiertes Aryl oder Heteroaryl steht,

sowie ihre pharmakologisch unbedenklichen Salze mit anorganischen oder organischen Säuren.

Es wurde gefunden, daß man 6,7-Dihydro-5H,13H-chinazolino[3,2-a][1,4]benzodiazepin-5,13-dione der Formel (I) erhält, wenn man 2-Alkyl-3-aryl-4H-chinazolin-4-one der allgemeinen Formel (II)

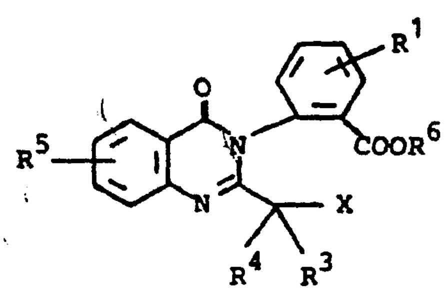

(II)

in welcher

$R^3$ und $R^4$ die oben angegebene Bedeutung haben,

$R^1$ und $R^5$ für 1, 2, 3 oder 4 Stubstituenten aus der Gruppe Wasserstoff, Alkyl, Hydroxy, Acyloxy, Alkoxy, Nitro, Dialkylamino, Acylamino, Acylalkylamino, Alkoxycarbonylamino, Halogen, Trifluormethyl, Cyan, Alkoxycarbonyl, Dialkylaminocarbonyl, Aminosulfonyl, Alkylaminosulfonyl, Dialkylaminosulfonyl und Dialkylaminocarbonylamino steht,

$R^6$ für Wasserstoff, Alkyl, Aralkyl oder Aryl steht und

X für eine leicht nucleophil substituierbare Abgangsgruppe wie Halogen oder Alkylsulfo, Aralkylsulfo oder Arylsulfo steht,

mit Ammoniak oder primären Aminen der allgemeinen Formel (III)

$$H_2N-R^2 \qquad (III)$$

in welcher

$R^2$ für gegebenenfalls substituiertes Alkyl, Aralkyl, Heterocycloalkyl oder Aryl steht,

oder mit deren Salzen mit anorganischen oder organischen Säuren oder Basen

in Gegenwart von inerten organischen Lösungsvermittlern und gegebenenfalls in Gegenwart von Säurebindern bei Temperaturen zwischen 20 und 250°C umsetzt

und gegebenenfalls anschließend in an sich bekannter Weise in einem weiteren Reaktionsschritt Nitrogruppen oder Acylaminogruppen als Substituenten $R^1$ und/oder $R^5$ in Aminogruppen oder

Acylalkylaminogruppen in Alkylaminogruppen oder

Alkoxycarbonylgruppen in Carboxygruppen oder

Alkoxycarbonylgruppen und/oder Cyangruppen in Aminocarbonyl- oder Alkylaminocarbonylgruppen oder

Aminogruppen in Alkylaminocarbonylaminogruppen überführt, oder im Falle der Umsetzung mit Ammoniak ($R^2$ = Wasserstoff) die NH-Gruppe mit Carbonsäure-, Sulfonsäurechloriden oder Kohlensäureesterchloriden oder mit Carbonsäureanhydriden oder mit Pyrokohlensäureestern nachträglich acyliert.

Die Umsetzung der 2-Alkyl-3-aryl-4H-chinazolin-4-one der Formel (II) mit Ammoniak oder primären Aminen der Formel (III) zu den 6,7-Dihydro-5H,13H-chinazolino[3,2-a][1,4]benzodiazepin-5,13-dionen der Formel (I) verläuft in 2 Reaktionsschritten über die Zwischenstufen IV und/oder V, welche gegebenenfalls isoliert werden können:

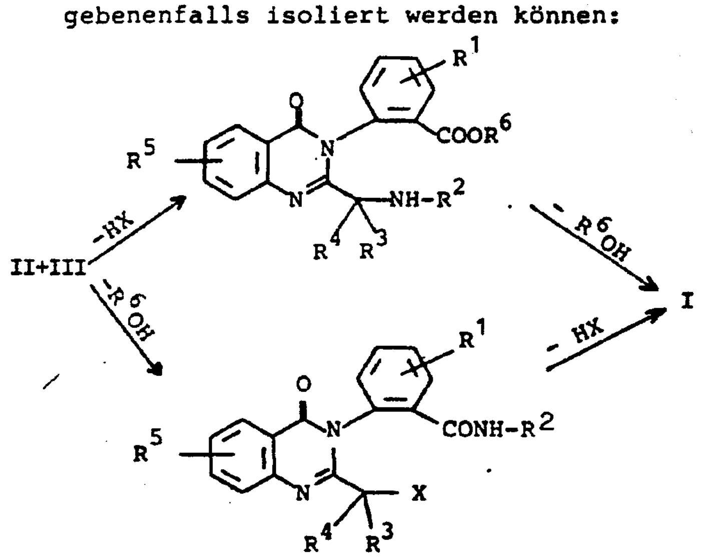


(IV)

(V)

Die erfindungsgemäßen Benzodiazepinderivate der Formel (I) zeigen überraschenderweise sehr starke Wirkungen auf das Zentralnervensystem, insbesondere sehr gute cerebralantiischämische, antiamnetische, lern-, leistungs- und gedächnisverbessernde, tranquillisierende, analgetische und antipyretische Eigenschaften und stellen somit eine Bereicherung der Pharmazie dar.

In dem folgenden Reaktionsschema werden exemplarisch Umsetzungen einzelner erfindungsgemäß verwendbarer Reaktionspartner gezeigt:

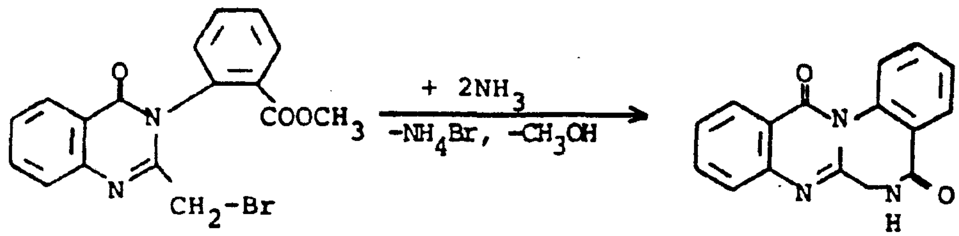


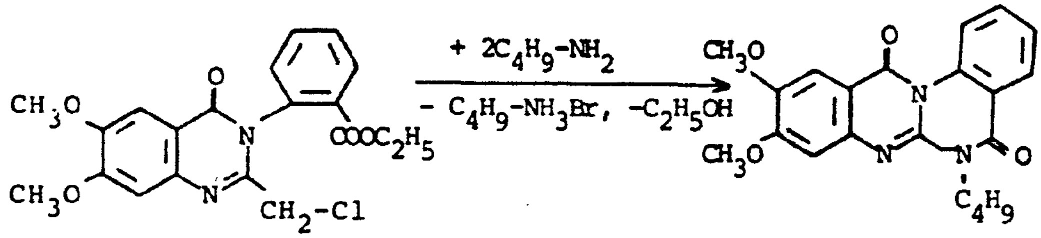


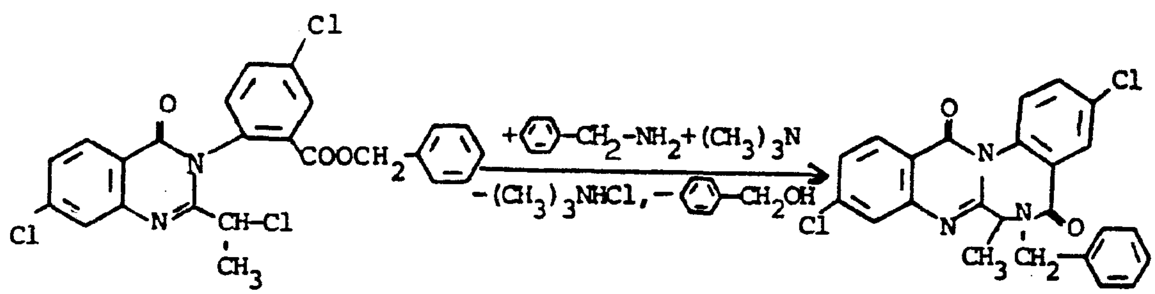


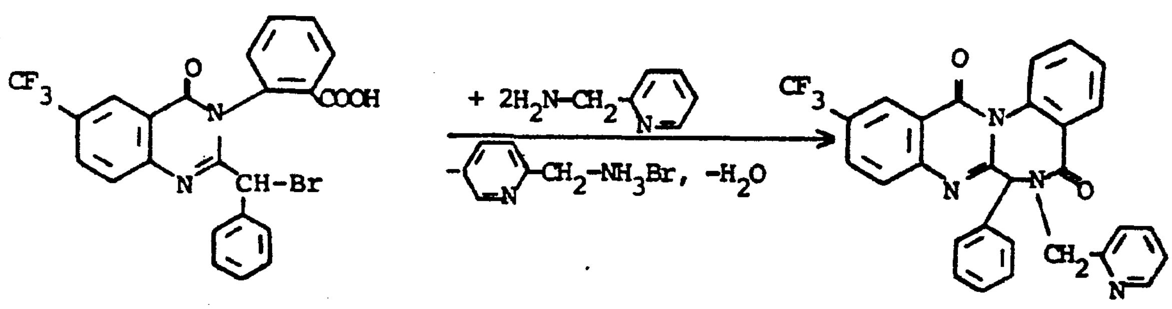

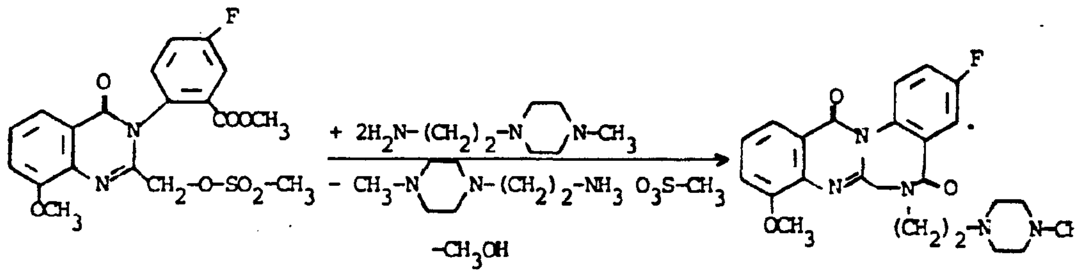


Beispiele für gegebenenfalls anschließende weitere Reaktionsschritte:

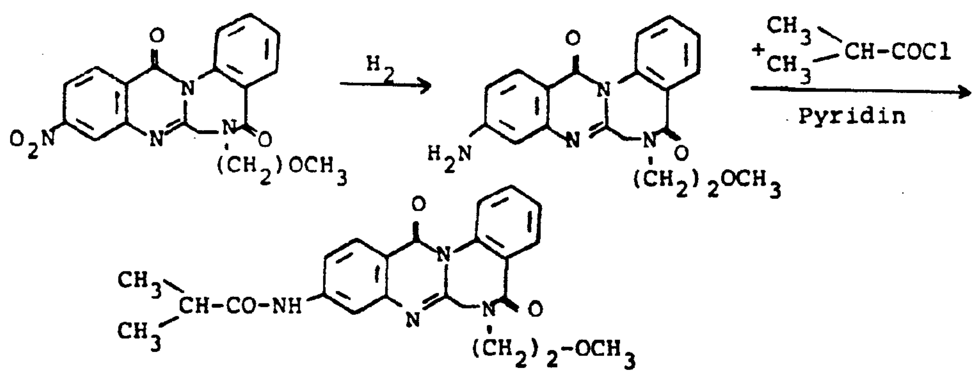


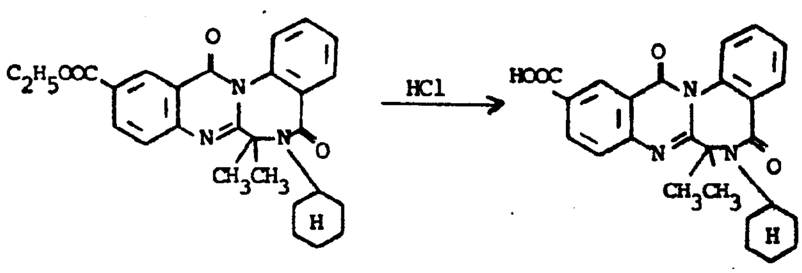

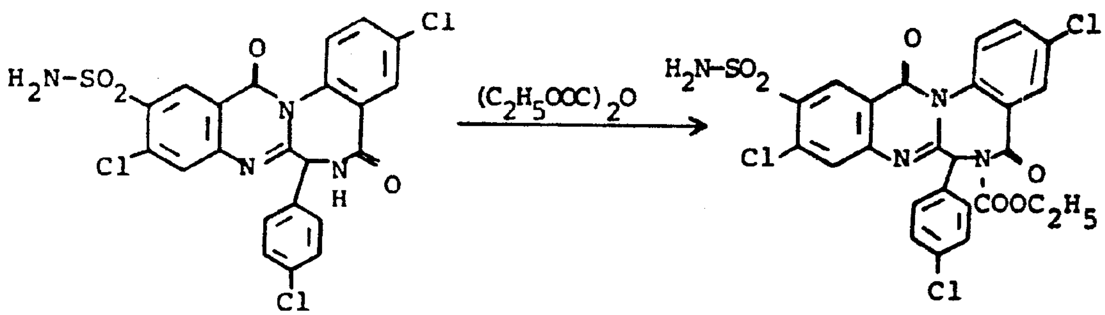
$(C_2H_5OOC)_2O$

In den Formeln (I) und (II) steht $R^1$ und $R^5$ vorzugsweise für ein oder zwei Substituenten aus der Gruppe Wasserstoff, Alkyl, Hydroxy, Alkoxy, Nitro, Amino, Dialkylamino, Acylamino, Alkoxycarbonylamino, Halogen, Trifluormethyl, Carboxy, Alkoxycarbonyl, Aminocarbonyl, Dialkylaminocarbonyl, Aminosulfonyl, Dialkylaminosulfonyl, wobei Halogen insbesondere für Fluor, Chlor und Brom steht und die genannten Alkyl-, Alkoxy- und Acylreste 1 bis 4 Kohlenstoffatome, insbesondere 1 oder 2 Kohlenstoffatome enthalten.

Die Alkylreste in Dialkylamino können auch in sich oder über ein Heteroatom aus der Gruppe O, S oder über eine NH- oder N-Alkylgruppe zu einem 4- bis 7-gliedrigen Ring geschlossen sein.

$R^3$ steht vorzugsweise für Wasserstoff und $R^4$ vorzugsweise für Wasserstoff, Alkyl mit 1 bis 16, insbesondere 1 bis 8, Kohlenstoffatomen, Aralkyl mit 1 bis 6 Kohlenstoffatomen im Alkylteil, insbesondere Benzyl oder Phenyläthyl, Aryl in Form von Phenyl und Heteroaryl in Form eines 5- bis 6-gliedrigen heterocyclischen Systems mit 1 bis 3 Heteroatomen aus der Gruppe Stickstoff, Sauerstoff und/oder Schwefel. Mögliche Substituenten im Arylteil von Aralkyl und von Aryl sind Halogen, insbesondere Brom und Chlor, Trifluormethyl, Hydroxy, Alkoxy und Nitro.

$R^6$ steht vorzugsweise für Alkyl mit 1 bis 2 Kohlenstoffatomen, für Benzyl und Phenyl und

X steht vorzugsweise für Halogen, insbesondere Brom und Chlor und für Methylsulfo, Phenylsulfo und 4-Methylphenylsulfo.

Die als Ausgangsstoffe zu verwendenden 2-Alkyl-3-aryl-4H-chinazolin-4-one der Formel II sind bekannt oder lassen sich nach bekannten Verfahren herstellen (vgl. J.Amer.Chem. Soc. 68 (1946), 542 / J.org.Chem. 14 (1949), 967 / J.Gen. Chem. (engl.Trans.) 30 (1960), 2333 und 34 (1964), 848 / Chem.Abstr. 52 (1958), 9147 / J.pr.Chem. (4) 14 (1961), 84 / Arzneimittelforschung 13 (1963) 688 / J.Med.Chem. 20 (1977), 379 / Synthesis 1977, 309).

Als Beispiele seien im einzelnen genannt:

2-Chlormethyl-3-(2-methoxycarbonyl-phenyl)-4H-chinazolin-4-on,
2-Brommethyl-3-(2-methoxycarbonyl-phenyl)-4H-chinazolin-4-on,
2-Chlormethyl-3-(2-äthoxycarbonyl-phenyl)-6-chlor-4H-chinazolin-4-on,
2-Brommethyl-3-(2-carboxy-phenyl)-4H-chinazolin-4-on,
2-Chlormethyl-3-(2-methoxycarbonyl-5-chlor-phenyl)-7-chlor-4H-chinazolin-4-on,
2-Methylsulfomethyl-3-(2-äthoxycarbonyl-phenyl)-6-trifluormethyl-4H-chinazolin-4-on,
2-Phenylsulfomethyl-3-(2-methoxycarbonyl-phenyl)-8-methoxy-4H-chinazolin-4-on,
2-Fluormethyl-3-(2-methoxycarbonyl-phenyl)-6-fluor-4H-chinazolin-4-on,
2-Chlormethyl-3-(2-äthoxycarbonyl-phenyl)-6,7-dimethoxy-4H-chinazolin-4-on,
2-Brommethyl-3-(2-butoxycarbonyl-phenyl)-6,7,8-trichlor-4H-chinazolin-4-on,
2-(1-Chloräthyl)-3-(2-methoxycarbonyl-phenyl)-6-dimethylamino-4H-chinazolin-4-on,
2-(α-Brombenzyl)-3-(2-carboxy-phenyl)-6-aminosulfonyl-7-chlor-4H-chinazolin-4-on,
2-(α-Bromfurfuryl)-3-(2-benzyloxycarbonyl-4,6-dichlor-phenyl)-4H-chinazolin-4-on,
2-(2-Brompropyl)-3-(2-methoxycarbonyl-4-trifluormethyl-phenyl)-6-nitro-4H-chinazolin-4-on.

In den Formeln (I) und (III) steht $R^2$ vorzugsweise für einen gegebenenfalls substituierten Alkylrest mit 1 bis 10, insbesondere 1 bis 6 Kohlenstoffatomen, einen gegebenenfalls substituierten Aralkylrest mit 1 bis 6 Kohlenstoffatomen im Alkylteil, insbesondere Benzyl oder Phenäthyl, einen gegebenenfalls substituierten Phenylrest, einen Heterocycloalkylrest mit 1 bis 6 Kohlenstoffatomen im Alkylteil und 4 bis 8 Ringgliedern sowie 1 bis 3 Heteroatome aus der Gruppe Stickstoff, Sauerstoff und/oder Schwefel im Heteroarylteil, dem auch ein Phenylring anelliert sein kann.

Mögliche Substituenten in $R^2$ = Alkyl sind Hydroxy-, Alkoxy-, Amino-, Alkylamino-, Dialkylamino-, Acylamino-, Alkoxycarbonylamino-, Trifluormethyl-, Cyan-, Carboxy-, Alkoxycarbonyl-, Aminocarbonyl-, Alkylaminocarbonyl-, Dialkylaminocarbonyl-, Alkylaminocarbonylamino- und Sulfogruppen, wobei Carboxy- und Sulfogruppen auch in Form von Salzen mit anorganischen und organischen Säuren vorliegen können. Die Alkylreste in Dialkylaminogruppen können auch in sich oder über ein Heteroatom aus der Gruppe Sauerstoff, Schwefel oder über eine NH-, N-Alkyl-, N-Acyl- oder N-Alkoxycarbonylgruppe zu einem 4- bis 8-gliedrigen Ring geschlossen sein. In den möglichen Substituenten erwähnte Alkyl-, Alkoxy- und Acylreste enthalten vorzugsweise 1 bis 4, insbesondere 1 bis 2 Kohlenstoffatome.

Mögliche Substituenten in $R^2$ = Aralkyl und Phenyl sind im

jeweiligen Arylteil Halogen, insbesondere Chlor und Brom, Trifluormethyl, Hydroxy, Alkoxy, Nitro und Alkyl.

Die als Ausgangsstoffe zu verwendenden primären Amine der Formel (III) sind bekannt oder lassen sich nach bekannten Verfahren herstellen.

Als Beispiele seien im einzelnen genannt:

Methylamin, Isobutylamin, 2-Äthylhexylamin, 4-Methylcyclohexylamin, 2-Norbornylamin, Allylamin, Propargylamin, Propylendiamin, 1-Amino-2-diäthylaminoäthan, Äthanolamin, 3-Äthoxypropylamin, Aminoessigsäureäthylester, Aminopropionitril, Aminoäthansulfonsäure-Natrium-Salz, Trifluormethyläthylamin, 2,4-Dichlorbenzylamin, 4-Chlorphenyläthylamin, 3-Äthoxyanilin, 4-Diäthylaminoanilin, 2-Aminomethylfuran, 4-Aminomethylpyridin, N-Aminoäthyl-N'-methyl-piperazin, N-Aminobutyl-thiomorpholin-1,1-dioxid, 1-Aminomethylisochinolin, 1-Aminopropyl-1,2,3-triazol.

In den Formeln (I), (II) und (III) und entsprechend auch (IV) und (V) steht Alkyl beispielsweise für Methyl, Äthyl, n- und iso-Propyl, n-, iso- und tert.-Butyl, n- und iso-Hexyl, Decyl, Hexadecyl, Allyl, Propargyl, Cyclohexyl.

Alkoxy steht beispielsweise für Methoxy, Äthoxy, n- und iso-Propoxy, n-, iso- und tert.-Butoxy, Allyloxy, Cyclobutyloxy.

Alkylamino und Dialkylamino steht beispielsweise für Methylamino, Dimethylamino, Äthylamino, Diäthylamino, Methyläthylamino, n- und iso-Propylamino, n-, iso- und tert.-Butylamino,

n- und iso-Dipropylamino, n-, iso- und tert.-Dibutylamino, Allylamino, Diallylamino - Dialkylamino auch für Pyrrolidino, Piperidino, Cyclohexylimino, Morpholino, Thiomorpholino, Piperazino, N-Methylpiperazino, Tetrahydrochinolino, 2-Methylindolino.

Acylamino steht beispielsweise für Formylamino, Acetylamino, Propionylamino, n- und iso-Butyrylamino, Valeroylamino, iso-Valeroylamino, Pivaloylamino.

Alkoxycarbonyl steht beispielsweise für Methoxycarbonyl, Äthoxycarbonyl, n- und iso-Propoxycarbonyl, n-, iso- und tert.-Butoxycarbonyl, Allyloxycarbonyl, Cyclohexyloxycarbonyl.

Aralkyl steht beispielsweise für Benzyl, 4-Chlorbenzyl, 2,6-Dichlorbenzyl, 3-Brombenzyl, 2-Methoxybenzyl, 3-Hydroxybenzyl, 2,4,6-Trimethylbenzyl, Phenyläthyl, 4-Chlorphenyläthyl, Phenylpropyl, Phenylbutyl.

Aryl steht beispielsweise für Phenyl, 2-Chlorphenyl, 2,4-Dichlorphenyl, 3-Nitrophenyl, 3-Trifluormethylphenyl, 2-Trifluormethyl-4-chlorphenyl, 2-Methylphenyl, 4-Äthylphenyl, 3,4-Dimethylphenyl, 3-Hydroxyphenyl, 2-Methoxyphenyl, 4-Äthoxyphenyl, 2-Methoxy-5-chlorphenyl, 2,5-Diäthoxyphenyl, 4-Aminosulfonylphenyl, 3-Dimethylaminophenyl, 3-Äthylaminophenyl, 3-Äthylamino-4-methylphenyl.

Heteroaryl steht beispielsweise für Furan, Thiophen, Pyrrol, Oxazol, Isoxazol, Thiazol, Pyrazol, Imidazol, Triazol, Pyridin, Pyrimidin, Thiazin, Indol, Benzimidazol, Benzoxazol, Benzthiazol, Indazol, Chinolin, Isochinolin, Chinazolin, Chinoxalin, Benzotriazin, Phthalazin.

Als Lösungsvermittler kommen alle organischen Lösemittel in Frage, die gegenüber den jeweiligen Reaktionspartnern inert sind. Hierzu gehören vorzugsweise aliphatische Alkohole wie Methanol, Äthanol, Isopropanol oder Butanol, Äther wie Tetrahydrofuran, Dioxan, Äthylenglykolmonomethyläther, Äthylenglykoldiäthyläther, Glykole wie Äthylenglykol, Propylenglykol, Diäthylenglykol und entsprechende Äther mit aliphatischen Alkoholen wie Diäthylenglykoldimethyläther, Kohlenwasserstoffe wie Ligroin, Toluol, Xylol, Tetralin, Halogenkohlenwasserstoffe wie Chloroform, Tetrachlorkohlenstoff, Chlorbenzol, Dichlorbenzole, Nitrile wie Acetonitril, Propionitril, Carbonsäureamide wie Dimethylformamid, Dimethylacetamid, heterocyclische Basen wie Pyridin, Picoline, Kollidine, Chinolin oder Isochinolin, ferner handelsübliche technische Gemische dieser Lösemittel.

Die Umsetzung kann bei Normaldruck aber auch bei erhöhtem Druck durchgeführt werden. Insbesondere bei der Verwendung von Ammoniak oder niedrig siedenden primären Aminen als Rekationspartner kann erhöhter Druck für die Umsetzung erforderlich sein.

Die Reaktionstemperaturen können in einem größeren Bereich variiert werden. Im allgemeinen arbeitet man bei Temperaturen zwischen 20 und 250°C, vorzugswiese zwischen 20 und 180°C, insbesondere zwischen 40 und 150°C.

Als Säurebindemittel können alle üblichen Säurebinder eingesetzt werden, Hierzu gehören anorganische Basen wie Alkali- und Erdalkalihydroxide, z.B. Natriumhydroxid, Kaliumhydroxid, Calciumhydroxid, Bariumhydroxid, Alkali- oder Erdalkalicarbonate wie Natriumcarbonat, Kaliumcarbonat, Calciumcarbonat, Natriumhydrogencarbonat, Kaliumhydrogencarbonat, Amide wie Natriumamid sowie organische Basen wie tertiäre Amine, z.B. Triäthylamin, N,N-Dimethylanilin, Pyridine, Chinoline und Isochinoline. Anstelle eines der üblichen Säurebindemittel kann man bei der Umsetzung auch in vorteilhafter Weise einen Überschuß des Reaktionspartners Ammoniak bzw. primäres Amin verwenden.

Bei der Durchführung des erfindungsgemäßen Verfahrens setzt man auf 1 Mol des 2-Alkyl-3-aryl-4H-chinazolin-4-ons der Formel (II) mindestens 1 Mol Ammoniak bzw. primäres Amin der Formel (III) und mindestens 1 Mol eines der genannten Säurebindemittel ein.

Im Verlauf der erfindungsgemäßen Umsetzung gehen die Ausgangsstoffe in der Regel ganz oder teilweise in Lösung, während die Endprodukte auskristallisieren. Die Abscheidung der Endprodukte kann durch Abkühlen und/oder durch Zugabe von Fällungsmitteln wie Wasser, niedere aliphatische Äther wie Diäthyläther oder Dibutyläther oder niedere aliphatische Kohlenwasserstoffe wie Petroläther oder Leichtbenzin beschleunigt werden.

Von besonderem Interesse sind 6,7-Dihydro-5H,13H-chinazolino[3,2-a][1,4]benzodiazepin-5,13-dione der Formel (I), in welcher

$R^1$ und $R^5$ gleich oder verschieden sind und jeweils für 1 oder 2 Substituenten aus der Gruppe Wasserstoff,

Halogen, Nitro, Cyano, Trifluormethyl, Amino, Alkyl mit 1 bis 4 Kohlenstoffatomen oder Alkoxy mit 1 bis 4 Kohlenstoffatomen stehen,

$R^2$ für Wasserstoff, Alkyl mit 1 bis 6 Kohlenstoffatomen, wobei die Alkylgruppe gegebenenfalls durch Phenyl, Pyridyl, Halogen, Alkoxy oder Dialkylamino substituiert sein kann und wobei die Alkyl- und Alkoxysubstituenten 1 bis 4 Kohlenstoffatome enthalten, oder für eine Alkoxycarbonylgruppe mit 1 bis 4 Kohlenstoffatomen im Alkoxyrest, steht und

$R^3$ und $R^4$ gleich oder verschieden sind und für Wasserstoff, Alkyl mit 1 bis 4 Kohlenstoffatomen oder Phenyl stehen.

Als neue Wirkstoffe seien im einzelnen genannt: 6,7-Dihydro-5H,13H-chinazolino[3,2-a][1,4]benzodiazepin-5,13-dion, 1-Chlor-6,7-dihydro-, 2-Chlor- ..., 3-Chlor-, 4-Chlor-, 9-Chlor-, 10-Chlor-, 11-Chlor-, 12-Chlor-, 1,3-Dichlor-, 1,4-Dichlor-, 9,11-Dichlor, 9,12-Dichlor-,1,2,3-Trichlor-, 9,10, 11-Trichlor-, 1,2,3,4-Tetrachlor-, 9,10,11,12-Tetrachlor-, 3-Brom-, 11-Brom-, 1,3-Dibrom-, 9,11-Dibrom-, 3-Fluor-, 11-Fluor-, 1,3-Dijod-, 9,11-Dijod-, 1-Trifluormethyl-, 2-Trifluormethyl-, 3-Trifluormethyl-, 4-Trifluormethyl-, 9-Trifluormethyl-, 10-Trifluormethyl-, 11-Trifluormethyl-, 12-Trifluormethyl-, 1-Methyl-, 2-Methyl-, 3-Methyl-, 9-Methyl-, 10-Methyl-, 11-Methyl-, 2,4-Dimethyl-, 10,12-Dimethyl-, 1-Äthyl-, 9-Äthyl-, 1-Methoxy-, 3-Methoxy-, 4-Methoxy-, 9-Methoxy-, 11-Methoxy-, 12-Methoxy-, 2,3-Dimethoxy-, 10,11-Dimethoxy-, 2-Äthoxy-, 10-Äthoxy-, 1-Hydroxy-, 2-Hydroxy-, 9-Hydroxy-, 10-Hydroxy-, 1-Nitro-, 2-Nitro-, 3-Nitro-, 4-Nitro-, 9-Nitro-, 10-Nitro-, 11-Nitro-, 12-Nitro-, 1,3-Dinitro-, 9,11-Dinitro-, 1-Nitro-3-chlor-, 9-Nitro-11-chlor-, 9,11-Dinitro-12-Chlor-, 1-Brom-3-nitro-, 9-Brom-11-nitro-, 1-Amino-, 2-Amino-, 3-Amino-, 4-Amino-, 9-Amino-, 10-Amino-, 11-Amino-, 12-Amino-, 1,3-Diamino-, 9,11-Diamino-, 10-Acetylamino-, 11-Acetylamino-, 2-Äthoxycarbonylamino-, 3-Äthoxycarbonylamino-, 11-Äthoxycarbonylamino-, 10-Dimethylamino-, 10-N-Äthyl-N-formyl-amino-, 10-Pyrrolidino-, 10-Piperidino-, 10-Morpholino-, 10-N-Methyl-piperazino-, 10-Äthylamino-, 10-Isopropoxycarbonylamino-, 2-Carboxy-, 3-Carboxy-, 4-Carboxy-, 2-Methoxycarbonyl-, 3-Äthoxycarbonyl-, 4-Butoxycarbonyl-, 2-Aminocarbonyl-, 3-Butylaminocarbonyl-, 4-Morpholinocarbonyl-, 10-Aminosulfonyl-, 11-Aminosulfonyl-, 10-Chlor-11-aminosulfonyl-, 11-Methylaminosulfonyl-, 11-Diäthylaminosulfonyl-, 9-Methoxy-11-chlor-12-methyl-, 9-Chlor-12-äthyl-, 3,10-Dichlor-, 3,11-Dichlor-, 2-Chlor-11-trifluormethyl-, 4-Chlor-10-trifluormethyl-, 3,11-Bistrifluormethyl-, 6-Methyl-, 6-Butyl-, 6-(2-Äthoxymethyl)-, 6-(3-Dimethylaminoäthyl)-, 6-(2-Diäthylaminoäthyl)-, 6-(2-Diäthylaminopropyl)-, 6-Äthoxycarbonylmethyl-, 6-Piperidinoäthyl-, 6-(4-Methylpiperidinoäthyl)-, 6-(N-Methylpiperazinopropyl)-, 6-Morpholinoäthyl-, 6-Benzyl-, 6-(4-Chlorbenzyl)-, 6-(2,4-Dichlorbenzyl)-, 6-(3-Hydroxybenzyl)-, 6-Phenyläthyl-, 6-(4-Methoxyphenyläthyl)-, 6-Acetyl-, 6-Äthoxycarbonyl-, 6-(Picolyl-2)-, 6-(Picolyl-4)-, 7-Methyl-, 7-Phenyl-, 7-(3-Chlorphenyl)-, 7-Thienyl-, 7-Methyl-7-(4-chlorphenyl)-, 7-(Furyl-2)-10-chlor-, 3-Chlor-6-benzyl-7-methyl-, 4-Chlor-6-äthyl-7-phenyl-11-trifluormethyl-6,7-dihydro-6H,13H-chinazolino/3,2-a7/1,47-benzodiazepin-5,13-dion.

Die neuen erfindungsgemäßen Verbindungen sind als Arzneimittel verwendbare Substanzen. Sie können je nach Verwendungsart verabreicht werden, beispielsweise intravenös, intramuskulär, subkutan, oral oder intravaginal.

Die neuen Wirkstoffe können in bekannter Weise in die üblichen Formulierungen übergeführt werden wie Tabletten, Kapseln, Dragees, Pillen, Granulate, Sirupe, Emulsionen, Suspensionen und Lösungen, unter Verwendung inerter, nichttoxischer, pharmazeutisch geeigneter Trägersubstanzen oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nichttoxische organische Lösungsmittel wie Paraffine (z.B. Erdölfraktionen), pflanzliche Öle (z.B. Erdnuß-/Sesamöl), Alkohole (z.B. Äthylalkohol, Glycerin), Glykole (z.B. Propylenglykol, Polyäthylenglykol), feste Tägerstoffe wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel wie nichtionogene und anionische Emulgatoren (z.B. Polyäthylen-Fettsäureester, Polyoxyäthylen-Fettalkoholäther, Alkylsulfonate und Arylsulfonate), Dis-

pergiermittel (z.B. Lignin, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral.

Im Falle der oralen Anwendung können Tabletten selbstverständlich außer den genannten Trägerstoffen auch Zusätze wie Natriumcitrat, Calciumcarbonat und Calciumphosphat zusammen mit verschiedenen Zuschlagstoffen wie Stärke, vorzugsweise Kartoffelstärke, Gelatine und dergleichen, enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden. Als besonders vorteilhaft für den Fall der parenteralen Anwendung hat sich die Tatsache herausgestellt, daß die erfindungsgemäßen Verbindungen in Wasser gut lösliche Salze zu bilden vermögen. Diese Salze werden erhalten, wenn man die erfindungsgemäßen Verbindungen in einem geeigneten Lösungsmittel mit der äquimolaren Menge einer nichttoxischen anorganischen oder organischen Base vereinigt. Als Beispiele seien genannt: Natronlauge, Kali-

lauge, Äthanolamin, Diäthanolamin, Triäthanolamin, Amino-tris-hydroxymethyl-methan, Glucosamin, N-Methylglucosamin. Derartige Salze können auch für die orale Anwendung der erfindungsgemäßen Verbindungen eine erhöhte Bedeutung besitzen, indem sie die Resorption je nach Wunsch beschleunigen oder verzögern. Als Beispiele seien außer den oben bereits erwähnten Salzen genannt: Magnesiumsalze, Calciumsalze, Aluminiumsalze und Eisensalze.

Im allgemeinen hat es sich als vorteilhaft erwiesen, bei parenteraler Applikation Mengen von etwa 0,05 bis 200 mg/kg, vorzugsweise etwa 0,1 bis 50 mg/kg Körpergewicht pro Tag zur Erzielung wirksamer Ergebnisse zu verabreichen, und bei oraler Applikation beträgt die Dosierung etwa 0,1 bis 500 mg/kg, vorzugsweise 0,5 bis 100 mg/kg Körpergewicht pro Tag.

Trotzdem kann es gegebenenfalls erforderlich sein, von den genanntenMengen abzuweichen, und zwar in Abhängigkeit vom Körßergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in wenigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen.

Diese Angaben gelten sowohl für die Anwendung der erfindungsgemäßen Verbindungen in der Veterinär- als auch in der Humanmedizin.

Herstellungsbeispiele

**Beispiel 1**

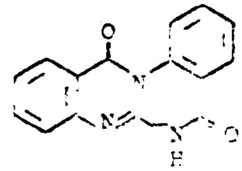

14,7 g (0,05 Mol) 2-Brommethyl-3-(2-methoxycarbonyl-phenyl)-4H-chinazolin-4-on und 25 ml flüssigen Ammoniaks in 100 ml Äthylenglykolmonomethyläther werden in einem Autoklaven 5 Stunden auf 100°C erhitzt. Nach dem Erkalten saugt man die farblosen Kristalle ab und wäscht mehrfach mit Methanol. Man erhält 6,1 g (44 % der Theorie) 6,7-Dihydro-5H,13H-chinazolino[3,2-a][1,4]benzodiazepin-5,13-dion. Schmelzpunkt: 313-315°C (aus Dimethylformamid).

a) Das Reaktionsprodukt des Beispiels 1 erhält man auch bei der Umsetzung von 32,9 g (0,1 Mol) 2-Chlormethyl-3-(2-methoxycarbonyl-phenyl)-4H-chinazolin-4-on und 80 ml flüssigen Ammoniaks in 200 ml Äthylenglykolmonomethyläther in einem Autoklaven in 5 Stunden bei 120°C in 69 %-iger Ausbeute.

2-Chlormethyl-3-(2-methoxycarbonyl-phenyl)-4H-chinazolin-4-on (Schmelzpunkt: 197-199°C, umkristallisiert aus Toluol) wurde analog J.pr.Chem. (4) 14 (1961), 84 aus N-Chloracetyl-anthranilsäure und Anthranilsäuremethylester hergestellt.

b) Das Reaktionsprodukt des Beispiels 1 erhält man auch durch 2 stündiges Einleiten von gasförmigem Ammoniak in eine Lösung von 0,05 Mol 2-Chlormethyl-2-(2-methoxycarbonyl-phenyl)-4H-chinazolin-4-on in 250 ml Dimethylformamid bei 20-25° und 12 stündiges Stehenlassen des Ansatzes in 81 %iger Ausbeute.

Beispiel 2

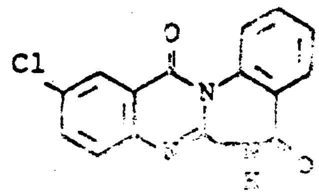

18,9 g (0,05 Mol) 2-Chlormethyl-3-(2-äthoxycarbonyl-phenyl)-6-chlor-4H-chinazolin-4-on und 50 ml flüssigen Ammoniaks in 150 ml Äthylenglykolmonoäthyläther werden in einem Autoklaven 3 Stunden auf 60°C erhitzt. Nach dem Erkalten saugt man die farblosen Kristalle ab und wäscht mehrfach mit Methanol. Man erhält 13 g (83 % der Theorie) 11-Chlor-6,7-dihydro-5H,13H-chinazolino[3,2-a][1,4]benzodiazepin-5,13-dion. Schmelzpunkt: 286-288°C (aus Dimethylformamid/Äthanol 1:1).

2-Chlormethyl-3-(2-äthoxycarbonyl-phenyl)-6-chlor-4H-chinazolin-4-on (Schmelzpunkt: 195-197°C, umkristallisiert aus Toluol) wurde analog J.Amer.Chem.Soc. 68 (1946), 542 aus N-Chloracetyl-5-chloranthranilsäure und Anthranilsäureäthylester hergestellt.

Beispiel 3

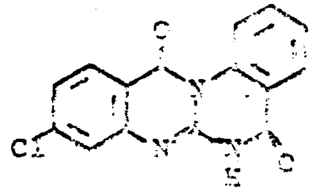

Analog Beispiel 1 a) erhält man aus 18 g (0,05 Mol) 2-Chlormethyl-3-(2-methoxycarbonyl-phenyl)-7-chlor-4H-chinazolin-4-on (Schmelzpunkt: 150-152°C aus Äthanol) und 50 ml flüssigen Ammoniaks in 150 ml Äthylenglykolmonomethyläther in 5

Stunden bei 80°C 10 g (65 % der Theorie) 10-Chlor-6,7-dihydro-5H,13H-chinazolino/3,2-a7/1,47benzodiazepin-5,13-dion. Schmelzpunkt: 290-292°C (aus Dimethylformamid).

**Beispiel 4**

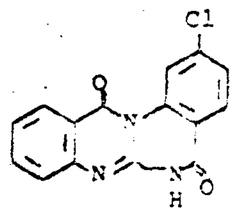

Analog Beispiel 1 a) erhält man aus 18 g (0,05 Mol) 2-Chlormethyl-3-(2-methoxycarbonyl-5-chlor-phenyl)-4H-chinazolin-4-on (Schmelzpunkt: 151-152°C aus Äthanol) und 50 ml flüssigen Ammoniaks in 150 ml Äthylenglykolmonomethyläther in 5 Stunden bei 80°C 9,5 g (61 % der Theorie) 2-Chlor-6,7-dihydro-5H-13H-chinazolino/3,2-a7/1,47benzodiazepin-5,13-dion. Schmelzpunkt: 330-333°C (aus Dimethylformamid).

**Beispiel 5**

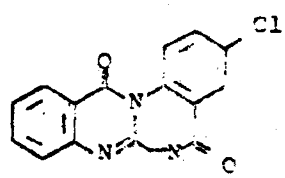

Analog Beispiel 1 a) erhält man aus 18 g (0,05 Mol) 2-Chlormethyl-3-(2-methoxycarbonyl-4-chlor-phenyl)-4H-chinazolin-4-on (Schmelzpunkt: 135-136°C aus Toluol) und 50 ml flüssigen Ammoniaks in 150 ml Äthylenglykolmonomethyläther in 5 Stunden bei 80°C 13 g (84 % der Theorie) 3-Chlor-6,7-dihydro-5H,13H-chinazolino/3,2-a7/1,47benzodiazepin-5,13-dion. Schmelzpunkt 294-296°C (aus Dimethylformamid).

Beispiel 6

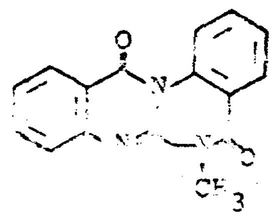

16,4 g (0,05 Mol) 2-Chlormethyl-3-(2-methoxycarbonyl-phenyl)-4H-chinazolin-4-on und 10 ml flüssigen Methylamins in 100 ml Äthylenglykolmonomethyläther werden in einem Autoklaven 2 Stunden auf 40°C erhitzt. Nach dem Erkalten saugt man die farblosen Kristalle ab und wäscht mehrfach mit Äthanol. Man erhält 12 g (82,3 % der Theorie) 6-Methyl-6,7-dihydro-5H,13H-chinazolino[3,2-a][1,4]benzodiazepin-5,13-dion. Schmelzpunkt: 204-206°C (aus Äthanol).

Beispiel 7

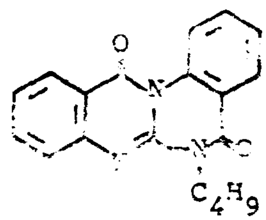

Analog Beispiel 6 erhält man aus 32,9 g (0,1 Mol) 2-Chlormethyl-3-(2-methoxycarbonyl-phenyl)-4H-chinazolin-4-on und 75 ml n-Butylamin in 200 ml Äthylenglykolmonomethyläther in 5 Stunden bei 120°C 7 g (21 % der Theorie) 6-n-Butyl-6,7-dihydro-5H,13H-chinazolino[3,2-a][1,4]benzodiazepin-5,13-dion. Schmelzpunkt: 125-127°C (aus Äthanol).

Beispiel 8

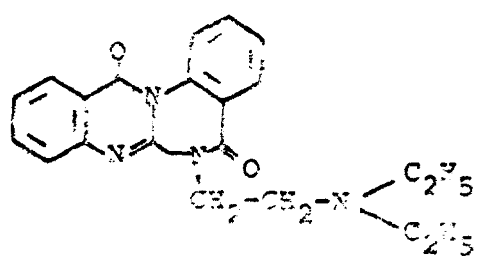

16,4 g (0,05 Mol) 2-Chlormethyl-3-(2-methoxycarbonyl-phenyl)-4H-chinazolin-4-on und 12,8 g (0,11 Mol) 1-Amino-2-dimethylamino-äthan in 100 ml Äthylenglykolmonomethyläther werden 5 Stunden auf 120°C erhitzt. Man verdampft das Lösungsmittel am Rotavapor und versetzt den Ölrückstand mit 50 ml Äthanol. Es entsteht eine klare Lösung und nach kurzer Zeit scheiden sich farblose Kristalle ab. Durch Absaugen und Waschen mit Äthanol und Wasser erhält man 12,4 g (66 % der Theorie) analysenreines 6-Diäthylaminoäthyl-6,7-dihydro-5H,13H-chinazolino[3,2-a][1,4]benzodiazepin-5,13-dion. Schmelzpunkt: 117-119°C.

Beispiel 9

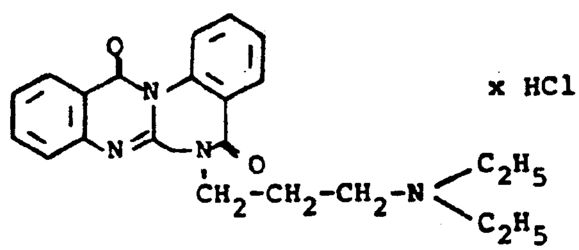

x HCl

16.4 g (0,05 Mol) 2-Chlormethyl-3-(2-methoxycarbonyl-phenyl)-4H-chinazolin-4-on und 14,3 g (0,11 Mol) 1-Amino-3-diäthylamino-propan in 100 ml Äthylenglykolmonomethyläther werden 5 Stunden auf 120°C erhitzt. Man verdampft das Lösungsmittel am Rotavapor und versetzt den Ölrückstand mit 50 ml Wasser und 100 ml Chloroform. Nach Abtrennen der Chloroformphase im Scheidetrichter schüttelt man diese mit Wasser, verdünnter Natronlauge und nochmals mit Wasser aus, trocknet über Natriumsulfat und verdampft das Chloroform am Rotavapor. Den Ölrückstand löst man in Äthanol und leitet bis zur Sättigung Chlorwasserstoff ein. Dabei kristallisiert analysenreines 6-Diäthylaminopropyl-6,7-dihydro-5H,13H-chinazolino[3,2-a][1,4]benzodiazepin-5,13-dion-hydrochlorid aus (15,1 g, 71 % der Theorie). Schmelzpunkt: 237-238°C.

Beispiel 10

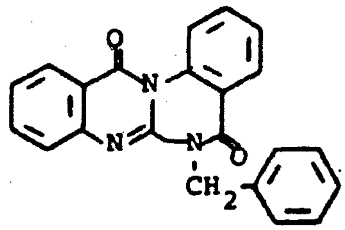

Analog Beispiel 1 a) erhält man aus 30 g (0,091 Mol) 2-Chlormethyl-3-(2-methoxycarbonyl-phenyl)-4H-chinazolin-4-on und 50 ml Benzylamin in 200 ml Äthylenglykolmonomethyläther in 5 Stunden bei 120°C 29,5 g (88 % der Theorie) 6-Benzyl-6,7-dihydro-5H,13H-chinazolino[3,2-a][1,4]benzodiazepin-5,13-dion. Schmelzpunkt: 171-173°C.

Beispiel 11

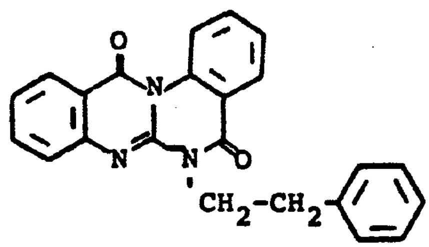

16,4 g (0,05 Mol) 2-Chlormethyl-3-(2-methoxycarbonyl-phenyl)-4H-chinazolin-4-on und 12,1 g (0,1 Mol) 2-Phenyläthylamin werden in 100 ml Äthylenglykolmonomethyläther 5 Stunden auf 100°C erhitzt. Man verdampft das Lösungsmittel am Rotavapor und versetzt den halbfesten Rückstand mit 100 ml Wasser. Die farblosen Kristalle werden abgesaugt und mit heißem Methanol gewaschen. Man erhält 5,8 g (30,5 % der Theorie) 6-(2-Phenyläthyl)-6,7-dihydro-5H,13H-chinazolino[3,2-a][1,4]benzodiazepin-5,13-dion. Schmelzpunkt: 208-210°C.

**Beispiel 12**

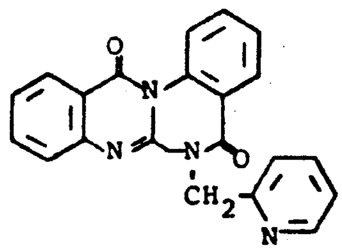

16,4 g (0,05 Mol) 2-Chlormethyl-3-(2-methoxycarbonyl-phenyl)-4H-chinazolin-4-on und 12 g (0,11 Mol) 2-Aminomethylpyridin in 100 ml Äthylenglykolmonomethyläther werden 5 Stunden auf 120°C erhitzt. Man verdampft das Lösungsmittel am Rotavapor und kristallisiert den festen Rückstand aus Methanol um. Nach dem Absaugen wäscht man mit Methanol und Wasser, wobei 10 g (54,5 % der Theorie) 6-(Pyridyl-(2)-methyl)-6,7-dihydro-5H,13H-chinazolino[3,2-a][1,4]benzodiazepin-5,13-dion erhalten werden. Schmelzpunkt: 202-203°C.

**Beispiel 13**

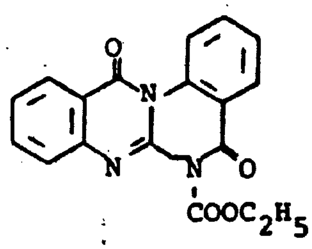

8 g (0,029 Mol) des Reaktionsproduktes aus Beispiel 1 werden in 30 ml Pyrokohlensäure-diäthylester 15 Minuten auf 120°C erhitzt, wobei man das sich bildende Äthanol laufend über einen absteigenden Kühler abdestilliert. Man verdampft Äthanol und überschüssiges Diäthylpyrocarbonat am Rotavapor und fügt zu dem Ölrückstand erneut 50 ml Äthanol. Es bilden sich farblose Kristalle, die nach dem Absaugen mit Äthanol gewaschen werden. Man erhält 6,4 g (63,5 % der Theorie) 6-Äthoxycarbonyl-6,7-dihydro-5H,13H-chinazolino[3,2-a][1,4]benzodiazepin-5,13-dion. Schmelzpunkt: 185-187°C.

Beispiel 14

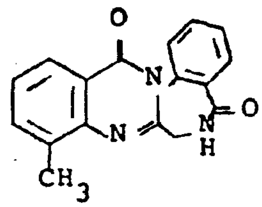

Analog Beispiel 1 a) erhält man aus 0,05 Mol 2-Chlormethyl-3-(2-methoxycarbonyl-phenyl)-8-methyl-4H-chinazolin-4-on (Schmp. 206 - 208°) und 50 ml flüssigen Ammoniaks in 150 ml Äthylenglykoldiäthyläther in 3 Stunden bei 60° 76 % d. Th. 9-Methyl-A.*) Schmp. 273 - 274° (aus n-Butanol).

*) Die in den meisten Beispielen verwendete Abkürzung A lautet: 6,7-dihydro-5H, 13H-chinazolino 3,2-a 1,4 benzodiazepin-5,13-dion

Beispiel 15

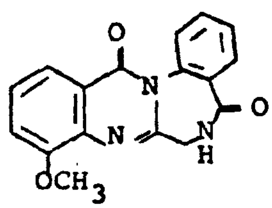

Analog Beispiel 1 a) erhält man aus 0,05 Mol 2-Chlormethyl-3-(2-methoxycarbonyl-phenyl)-8-methoxy-4H-chinazolin-4-on (Schmp. 208 - 210°) und 50 ml flüssigen Ammoniaks in 150 ml n-Butanol in 3 Stunden bei 60° 52 % d. Th. 9-Methoxy-A.* Schmp. 290 - 292° (aus n-Butanol).

Beispiel 16

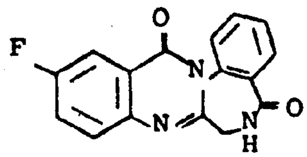

Analog Beispiel 1 a) erhält man aus 0,135 Mol 2-Chlormethyl-3-(2-methoxycarbonyl-phenyl)-6-fluor-4H-chinazolin-4-on (Schmp. 213 - 215°) in 400 ml Äthylenglykol und 140 ml flüssigen Ammoniaks in 3 Stunden bei 60° 85 % d. Th. 11-Fluor-A. Schmp. 275 - 277° (aus Äthylenglykolmonomethyläther).

Beispiel 17

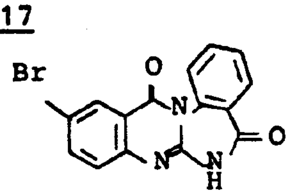

Analog Beispiel 1 a) erhält man aus 0,05 Mol 2-Chlormethyl-3-(2-methoxycarbonyl-phenyl)-6-brom-4H-chinazolin-4-on (Schmp. 235 - 237°) und 50 ml flüssigen Ammoniaks in 150 ml Pyridin in 5 Stunden bei 60° 88 % d. Th. 11-Brom-A. Schmp. 300 - 303°.

Beispiel 18

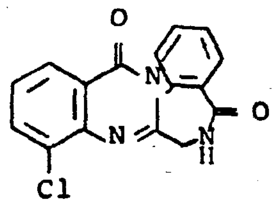

Analog Beispiel 1 a) erhält man aus 0,025 Mol 2-Chlormethyl-3-(2-methoxycarbonyl-phenyl)-8-chlor-4H-chinazolin-4-on (Schmp. 205 - 208°) und 20 ml flüssigen Ammoniaks in 50 ml Äthylenglykolmonoäthyläther in 3 Stunden bei 50° 71 % d. Th. 9-Chlor-A. Schmp. 255 - 258°.

Beispiel 19

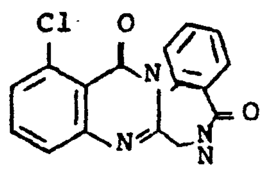

Analog Beispiel 1 a) erhält man aus 0,05 Mol 2-Chlormethyl-3-(2-methoxycarbonyl-phenyl)-5-chlor-4H-chinazolin-4-on (Schmp. 177 - 178°) und 50 ml flüssigen Ammoniaks in 150 ml Äthylenglykoldimethyläther in 3 Stunden bei 60° 60 % d. Th. 12-Chlor-A. Schmp. 308 - 310° (aus Dimethylformamid).

Beispiel 20

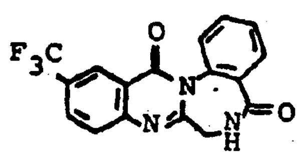

Analog Beispiel 1 a) erhält man aus 0,05 Mol 2-Chlormethyl-3-(2-methoxycarbonyl-phenyl)-6-trifluormethyl-4H-chinazolin-4-on (Schmp. 169 - 170°) und 50 ml flüssigen Ammoniaks in 150 ml Dioxan in 3 Stunden bei 50° 36 % d.Th. 11-Trifluormethyl-A. Schmp. 190 - 192° (aus Toluol).

Beispiel 21

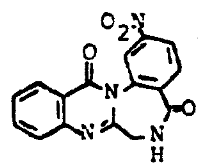

Analog Beispiel 1 a) erhält man aus 0,05 Mol 2-Chlormethyl-3-(2-methoxycarbonyl-5-nitro-phenyl)-4H-chinazolin-4-on (Schmp. 158 - 159°) und 50 ml flüssigen Ammoniaks in 150 ml o-Dichlorbenzol in 3 Stunden bei 60° 35 % d. Th. 2-Nitro-A. Schmp. 276 - 277° Zers. (aus Eisessig).

Beispiel 22

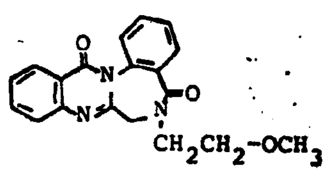

Analog Beispiel 8 erhält man aus 0,05 Mol 2-Methoxymethyl-3-(2-methoxycarbonyl-phenyl)-4H-chinazolin-4-on (Schmp. 134-137°, hergestellt aus 2-Brommethyl-3-(2-methoxycarbonyl-phenyl)-4H-chinazolin-4-on und Methanol analog J. Med. Chem. 20, 379 (1977)) und 20 ml 2-Methoxyäthylamin in 100 ml Äthanol in

3 Stunden bei 50° 40 % d.Th. 6-Methoxyäthyl-A. Schmp. 127 - 130° (aus Äthanol).

Beispiel 23

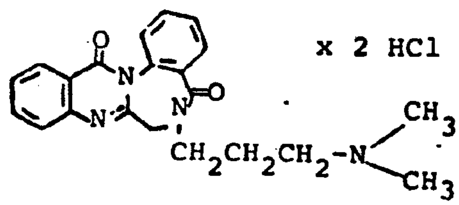

x 2 HCl

Analog Beispiel 9 erhält man aus 0,05 Mol 2-Chlormethyl-3-(2-äthoxycarbonyl-phenyl)-4H-chinazolin-4-on und 10 g 3-Dimethylaminopropylamin in 100 ml Äthylenglykolmonoäthyläther in 5 Stunden bei 120° 67 % d. Th. 6-Dimethylaminopropyl-A-dihydrochlorid. Schmp. 198 - 200° (aus Äthanol).

Beispiel 24

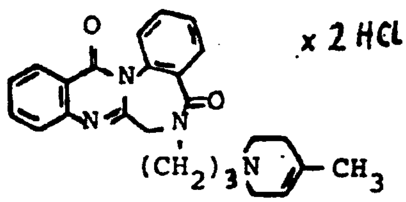

x 2 HCl

Analog Beispiel 9 erhält man aus 0,05 Mol 2-Chlormethyl-3-(2-äthoxycarbonyl-phenyl)-4H-chinazolin-4-on und 17 g N-Aminopropyl-4-methyl-1,2,5,6-tetrahydro-pyridin in 100 ml Tetralin in 5 Stunden bei 120° 45 % d. T. 6-(4-Methyl-1,2,4,6-tetrahydro-pyridino-propyl)-A-dihydrochlorid. Schmp. 112-115° (aus Äthanol).

Beispiel 25

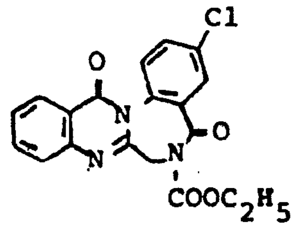

Analog Beispiel 13 erhält man aus 0,05 Mol des Reaktionsproduktes aus Beispiel 5 und 60 ml Pyrokohlensäurediäthylester in 20 Minuten bei 120-130° 80 % d. Th. 6-Äthoxycarbonyl-3-chlor-A. Schmp. 189 - 191° (aus Äthanol).

Beispiel 26

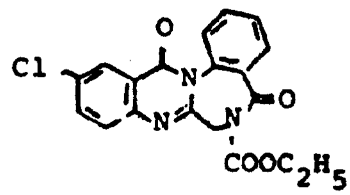

Analog Beispiel 13 erhält man aus 0,015 Mol des Reaktionsproduktes aus Beispiel 2 und 20 ml Pyrokohlensäurediäthylester in 30 Minuten bei 130 - 135° 94 % d. Th. 6-Äthoxycarbonyl-11-chlor-A. Schmp. 258 - 260° (aus Äthanol).

Beispiel 27

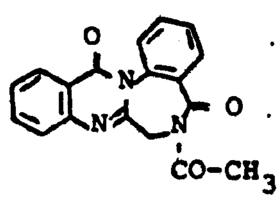

0,05 Mol des Reaktionsproduktes aus Beispiel 1 werden in 100 ml Essigsäureanhydrid und 10 ml Pyridin 8 Stunden auf 120° erhitzt. Nach dem Erkalten gießt man die klare Lösung auf Wasser und saugt die ausgefallenen Kristalle ab. Man erhält 44 % d. Th. 6-Acetyl-A. Schmp. 189 - 191° (aus Äthanol).

Beispiel 28

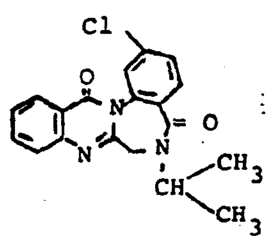

Analog Beispiel 1 a) erhält man aus 0,03 Mol 2-Chlormethyl-3-(2-methoxycarbonyl-5-chlor-phenyl)-4H-chinazolin-4-on und 25 ml Isopropylamin (65 %ige Lösung in Wasser) in 100 ml Isopropanol in 3 Stunden bei 50° 48 % d.Th. 2-Chlor-6-isopropyl-A. (Schmp. 193 - 195° (aus Äthanol).

Beispiel 29

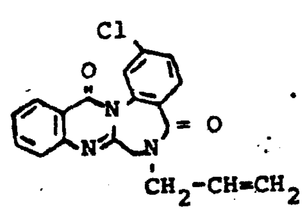

Analog Beispiel 1a) erhält man aus 0,03 Mol 2-Chlormethyl-3-(2-methoxycarbonyl-5-chlor-phenyl)-4H-chinazolin-4-on und 15 ml Allylamin in 100 ml Toluol in 3 Stunden bei 50° 89 % der Theorie 6-Allyl-2-chlor-A. Schmp. 155-157°.

Beispiel 30

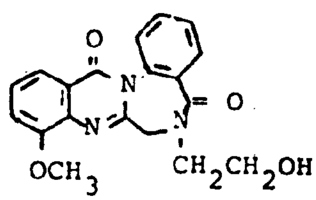

Analog Beispiel 8 erhält man aus 0,035 Mol 2-Chlormethyl-3-(2-methoxycarbonyl-phenyl)-8-methoxy-4H-chinazolin-4-on und 15 ml Äthanolamin in 100 ml Propylenglykol in 5 Stunden bei 80° 80,5 % d.Th. 6-Hydroxyäthyl-9-methoxy-A. Schmp. 233-235°.

Beispiel 31

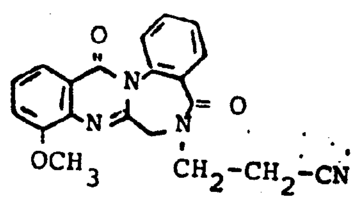

Analog Beispiel 8 erhält man aus 0,035 Mol 2-Chlormethyl-3-(2-methoxycarbonyl-phenyl)-8-methoxy-4H-chinazolin-4-on und 15 ml Cyanäthylamin in 100 ml Äthanol in 5 Stunden bei 80° 51 % d.Th. 6-Cyanäthyl-9-methoxy-A. Schmp. 198-200° (aus Äthanol).

Beispiel 32

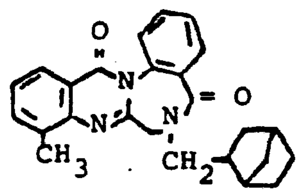

Analog Beispiel 8 erhält man aus 0,03 Mol 2-Chlormethyl-3-(2-methoxycarbonyl-phenyl)-8-methyl-4H-chinazolin-4-on und 15 ml 2-Norbornylamin in 100 ml Äthylenglykolmonomethyläther in 5 Stunden bei 80° 62 % d.Th. 6-Norbornyl-(2)-9-methyl-A. Schmp. 168-169° (aus Methanol).

Beispiel 33

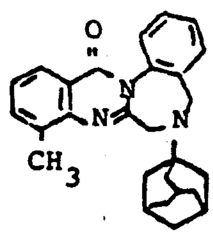

Analog Beispiel 8 erhält man aus 0,03 Mol 2-Chlormethyl-3-(2-methoxycarbonyl-phenyl)-8-methyl-4H-chinazolin-4-on und 0,07 Mol 1-Aminoadamantan in 100 ml Äthylenglykolmonoäthyläther in 5 Stunden bei 125-130° 90 % d.Th. 6-Adamantyl-(1 9-methyl-A. Schmp. 204-206°.

Beispiel 34

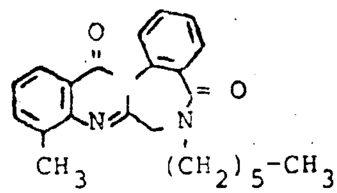

Analog Beispiel 8 erhält man aus 0,03 Mol 2-Chlormethyl-3-(2-methoxycarbonyl-phenyl)-8-methyl-4H-chinazolin-4-on und 15 ml n-Hexylamin in 100 ml n-Butanol in 5 Stunden bei 80° 88,5 % d.Th. 6-Hexyl-9-methyl-A. Schmp. 138-139°.

Beispiel 35

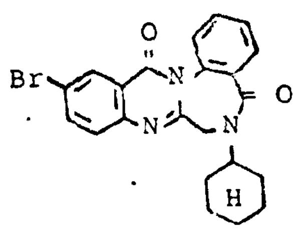

Analog Beispiel 8 erhält man aus 0,05 Mol 2-Chlormethyl-3-(2-methoxycarbonyl-phenyl)-6-brom-4H-chinazolin-4-on und 20 ml Cyclohexylamin in 100 ml Äthylenglykol in 5 Stunden bei 125° 43 % d.Th. 11-Brom-6-cyclohexyl-A. Schmp. 276-279°.

Beispiel 36

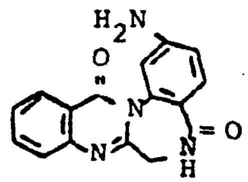

0,06 Mol des Reaktionsproduktes aus Beispiel 21 werden in 150 ml Dimethylformamid in Gegenwart von 5 g Raney-Nickel bei 60° hydriert. Nach dem Abfiltrieren des Katalysators wird die Lösung eingedampft (Rotavapor) und der Rückstand mit Äthylenglykolmonomethyläther heiß extrahiert. Beim Abkühlen kristallisiert analysenreines 2-Amino-A aus. Ausbeute 50 % d.Th., Schmp. > 340°.

Beispiel 37

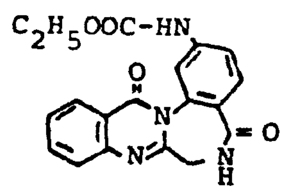

0,015 Mol des Reaktionsproduktes aus Beispiel 36 werden in 100 ml Äthanol mit 10 g Pyrokohlensäurediäthylester 12 Stunden auf 80° erhitzt. Nach Abkühlen, Absaugen und Waschen mit Äthanol erhält man 89 % d.Th. 2-Äthoxycarbonylamino-A. Schmp. 335° Zers.

Patentansprüche

1) 6,7-Dihydro-5H,13H-chinazolino[3,2-a][1,4]benzodiazepin-5,13-dione der allgemeinen Formel (I)

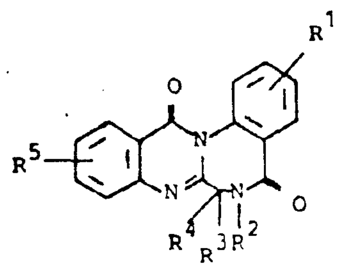

(I)

in welcher

$R^1$ und $R^5$ für 1, 2, 3 oder 4 Substituenten aus der Gruppe Wasserstoff, Alkyl, Hydroxy, Acyloxy, Alkoxy, Nitro, Amino, Alkylamino, Dialkylamino, Acylamino, Acylalkylamino, Alkoxycarbonylamino, Halogen, Trifluormethyl, Cyan, Carboxy, Alkoxycarbonyl, Aminocarbonyl, Alkylaminocarbonyl, Dialkylaminocarbonyl, Aminosulfonyl, Alkylaminosulfonyl, Dialkylaminosulfonyl, Alkylaminocarbonylamino und Dialkylaminocarbonylamino steht,

$R^2$ für Wasserstoff, gegebenenfalls substituiertes Alkyl, Aralkyl, Aryl oder für Heterocycloalkyl, Acyl oder Alkoxycarbonyl steht und

$R^3$ und $R^4$ für gleiche oder verschiedene Substituenten aus der Gruppe Wasserstoff, gegebenenfalls substituiertes Alkyl, gegebenenfalls im Arylteil substituiertes Aralkyl, gegebenenfalls substituiertes Aryl oder Heteroaryl steht,

sowie ihre pharmakologisch unbedenklichen Salze mit anorganischen oder organischen Säuren.

2) Verfahren zur Herstellung von 6,7-Dihydro-5H,13H-chinazolino[3,2-a][1,4]benzodiazepin-5,13-dionen der allgemeinen Formel (I), dadurch gekennzeichnet, daß man 2-Alkyl-3-aryl-4H-chinazolin-4-one der allgemeinen Formel (II)

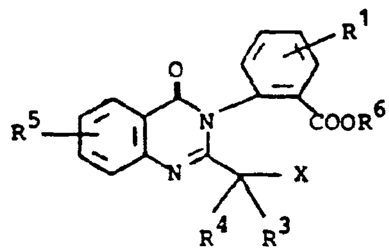

(II)

in welcher

| | |
|---|---|
| $R^3$ und $R^4$ | die oben angegebene Bedeutung haben, |
| $R^1$ und $R^5$ | für 1, 2, 3 oder 4 Stubstituenten aus der Gruppe Wasserstoff, Alkyl, Hydroxy, Acyloxy, Alkoxy, Nitro, Dialkylamino, Acylamino, Acylalkylamino, Alkoxycarbonylamino, Halogen, Trifluormethyl, Cyan, Alkoxycarbonyl, Dialkylaminocarbonyl, Aminosulfonyl, Alkylaminosulfonyl, Dialkylaminosulfonyl und Dialkylaminocarbonylamino steht, |
| $R^6$ | für Wasserstoff, Alkyl, Aralkyl oder Aryl steht und |
| X | für eine leicht nucleophil substituierbare Abgangsgruppe wie Halogen oder Alkylsulfo, Aralkylsulfo oder Arylsulfo steht, |

mit Ammoniak oder primären Aminen der allgemeinen Formel (III)

$$H_2N-R^2 \qquad (III)$$

in welcher

| | |
|---|---|
| $R^2$ | für gegebenenfalls substituiertes Alkyl, Aralkyl, Heterocycloalkyl oder Aryl steht, |

oder mit deren Salzen mit anorganischen oder organischen Säuren oder Basen

in Gegenwart von inerten organischen Lösungsvermittlern und gegebenenfalls in Gegenwart von Säurebindern bei Temperaturen zwischen 20 und 250°C umsetzt

und gegebenenfalls anschließend in an sich bekannter Weise in einem weiteren Reaktionsschritt Nitrogruppen oder Acylaminogruppen als Substituenten $R^1$ und/oder $R^5$ in Aminogruppen oder

Acylalkylaminogruppen in Alkylaminogruppen oder

Alkoxycarbonylgruppen in Carboxygruppen oder

Alkoxycarbonylgruppen und/oder Cyangruppen in Aminocarbonyl- oder Alkylaminocarbonylgruppen oder

Aminogruppen in Alkylaminocarbonylaminogruppen überführt, oder

im Falle der Umsetzung mit Ammoniak ($R^2$ = Wasserstoff) die NH-Gruppe mit Carbonsäure-, Sulfonsäure- oder Kohlensäureesterchloriden oder mit Carbonsäureanhydriden oder mit Pyrokohlensäureestern nachträglich acyliert.

3) Verbindungen gemäß Formel (I) in Anspruch 1, in welcher die Substituenten

$R^1$ und $R^5$ gleich oder verschieden sind und jeweils für 1 oder 2 Substituenten aus der Gruppe Wasserstoff, Halogen, Nitro, Cyano, Trifluormethyl, Amino, Alkyl mit 1 bis 4 Kohlenstoffatomen oder Alkoxy mit 1 bis 4 Kohlenstoffatomen stehen,

$R^2$ für Wasserstoff, Alkyl mit 1 bis 6 Kohlenstoffatomen, wobei die Alkylgruppe gegebenenfalls durch Phenyl, Pyridyl, Halogen, Alkoxy oder Dialkylamino substituiert sein kann und wobei die Alkyl- und Alkoxysubstituenten 1 bis 4 Kohlenstoffatome enthalten, oder für eine Alkoxycarbonylgruppe mit 1 bis 4 Kohlenstoffatomen im Alkoxyrest, steht und

$R^3$ und $R^4$ gleich oder verschieden sind und für Wasserstoff, Alkyl mit 1 bis 4 Kohlenstoffatomen oder Phenyl stehen.

4) Arzneimittel enthaltend Verbindungen gemäß Anspruch 1.

5) Verfahren zur Herstellung von Arzneimitteln, dadurch gekennzeichnet, daß man mindestens ein 6,7-Dihydro-5H,13H-chinazolino[3,2-a][1,4]benzodazepin-5,13-dion gemäß Anspruch 1 mit üblichen Hilfs- und Trägerstoffen vermischt.

6) Verwendung von 6,7-Dihydro-5H,13H-chinazolino[3,2-a][1,4]benzodiazepin-5,13-dionen gemäß Anspruch 1 zur Behandlung des Zentralnervensystems.

0 000 538

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 78 10 0425

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| A | US - A - 3 280 117 (SANDOZ)<br>* Spalte 1, Zeilen 7-33 *<br>---- | 1,4 | C 07 D 487/04<br>A 61 K 31/55//<br>C 07 D 239/91<br>(C 07 D 487/04<br>C 07 D 243/00<br>C 07 D 239/00) |
| | | | RECHERCHIERTE SACHGEBIETE (Int. Cl.²) |
| | | | C 07 D 487/04<br>A 61 K 31/55//<br>(C 07 D 487/04<br>C 07 D 243/00<br>C 07 D 239/00) |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-5

Unvollständig recherchierte Patentansprüche: 6

Nicht recherchierte Patentansprüche:

Grund für die Beschränkung der Recherche: Verfahren zur chirurgischen oder therapeutischen Behandlung des menschlichen oder tierischen Körpers.

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 26-10-1978 | ALFARO |

Europäisches
Patentamt

## GEBÜHRENPFLICHTIGE PATENTANSPRÜCHE

Die vorliegende europäische Patentanmeldung enthielt bei ihrer Einreichung mehr als zehn Patentansprüche.

☐ Alle Anspruchsgebühren wurden innerhalb der vorgeschriebenen Frist entrichtet. Der vorliegende europäische Recherchenbericht wurde für alle Patentansprüche erstellt.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt für die Anspruchsgebühren entrichtet wurden,

nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung; sie enthält mehrere Erfindungen oder Gruppen von Erfindungen,
nämlich:

☐ Alle weiteren Recherchengebühren wurden innerhalb der gesetzten Frist entrichtet. Der vorliegende europäische Recherchenbericht wurde für alle Patentansprüche erstellt.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind,

nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen,

nämlich Patentansprüche: